(19)

**Europäisches Patentamt**
**European Patent Office**
**Office européen des brevets**

(11) **EP 4 397 271 B1**

(12) **EUROPEAN PATENT SPECIFICATION**

(45) Date of publication and mention
of the grant of the patent:
**24.09.2025 Bulletin 2025/39**

(21) Application number: **24150155.0**

(22) Date of filing: **03.01.2024**

(51) International Patent Classification (IPC):
***A61B 34/20*** *(2016.01)*

(52) Cooperative Patent Classification (CPC):
**A61B 34/20; A61B 17/80; A61B 34/10;
A61B 34/30; A61B 34/76;** A61B 2034/105;
A61B 2034/108; A61B 2034/2051; A61B 2034/2055;
A61B 2034/2063; A61B 2034/2068

(54) **PATIENT REREGISTRATION SYSTEMS**

PATIENTENNEUREGISTRIERUNGSSYSTEME

SYSTÈMES DE RÉENREGISTREMENT DE PATIENT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.01.2023 US 202363478420 P**

(43) Date of publication of application:
**10.07.2024 Bulletin 2024/28**

(73) Proprietor: **Stryker Australia PTY LTD
Artarmon, NSW 1570 (AU)**

(72) Inventors:
• **WILLIAMSON, Tom
Brunswick, Virgin Islands 3056 (AU)**
• **HILL, David Gibson
Heathmont, Virgin Islands 3135 (AU)**
• **SWEENEY, Zachary
Melbourne, Virgin Islands 3051 (AU)**

• **BUEHNER, Ulrich
79211 Denzlingen (DE)**

(74) Representative: **ABG Intellectual Property Law,
S.L.
Avenida de Burgos, 16D
Edificio Euromor
28036 Madrid (ES)**

(56) References cited:
WO-A1-2021/207471    WO-A1-2021/243241
US-A1- 2017 252 169    US-A1- 2018 263 714

• **ELVIS C. S. CHEN: "Contact-less stylus for
surgical navigation: registration without
digitization", vol. 12, no. 7, 6 April 2017
(2017-04-06), DE, pages 1231 - 1241,
XP093159100, ISSN: 1861-6410, Retrieved from
the Internet <URL:https://link.springer.com/
content/pdf/10.1007/s11548-017-1576-7.pdf>
DOI: 10.1007/s11548-017-1576-7**

**Description**

BACKGROUND OF THE INVENTION

A significant advantage of image-guided surgery, such as robotic and computer-assisted surgery, is that it allows for very precise and accurate execution of a surgical plan. A surgical plan of this nature is typically developed virtually in a preoperative image coordinate space using a medical image of a patient's bone. Typically, the medical image is rendered in three-dimensions and positioned within the preoperative coordinate space so that certain operations, such as cutting, drilling, reaming, milling, and the like, can be performed on the virtual bone as would be desired during the surgical procedure.

During the surgical procedure, the preoperative coordinate space is correlated with an intraoperative coordinate space in a process called "registration" to ensure the virtual operations from the preoperative plan are executed precisely and accurately on the physical bone. This may be done by rigidly fixing a coordinate reference, such as an anatomy tracker, to the bone and using the coordinate reference's known position within the intraoperative coordinate space and relative to the bone to map the patient's bone onto the virtual bone. Image-guided surgery relies on accurate registration of the patient to the pre-operative image data to allow execution of the surgical plan. However, accurately registering the patient can be time-consuming process and is one of the most significant sources of error in image-guided procedures. Thus, even though image-guided surgery allows for very precise and accurate execution of a preoperative plan, its accuracy and precision are reliant on accurate registration.

After registration, any change in the position or orientation of the coordinate reference relative to the patient's actual anatomy during the procedure invalidates the existing registration thereby requiring the entire registration process to be repeated with no guarantee that the same transformation between coordinate spaces will be computed. Thus, the initial positioning of the coordinate reference on the patient's anatomy has been critical to image-guided surgery as incorrect placement may lead to collisions or interference between components (e.g., with the robotic arm) and issues with line-of-sight. Moreover, it has been critical for the reference tracker to not move relative to the bone once the procedure has started in order to minimize workflow disruption and prevent subsequent errors. These requirements have limited the viability of robotic or navigation assistance particularly in cases in which repositioning of the patient is desired or necessary due to limitations in access or reachability. Most robotic systems have a preferred working configuration and may not be able to perform some tasks if they fall outside of the robot's reachable workspace, while larger changes in position and orientation of tracking markers will likely lead to the aforementioned line of sight issues.

Therefore, there is a need for devices, systems, and methods for patient reregistration particularly for procedures that involve patient or robotic repositioning and in circumstances in which an initial registration is invalidated due to movement of the coordinate reference relative to the patient's bone. Additionally, there is a need to be quickly and easily detect an implant in a revision procedure for navigated or robotic explantation.

Document WO2021/243241A1 describes an implant for correcting a defect of a bone structure with navigation fiducials.

BRIEF SUMMARY OF THE INVENTION

The invention is defined in independent claim 1. Described herein are devices, systems, and methods for reregistration in image-guided surgery. In one aspect, an implant is designed to include integral registration features such that when the patient is moved, registration can be performed in relation to the implant for the execution of particular operations relative thereto. In another aspect, a registration block may be used in conjunction with a reference tracker so that, should the reference tracker be moved relative to the bone, the registration block can be used for reregistration thereby significantly reducing the time, complication, and possible errors that can occur by performing the entire registration process over from the beginning. In other words, registration builds up a chain of matrix transformations. If that chain of transformations is broken, such as through inadvertent movement of the reference tracker, for example, the broken link in that chain can be repaired by using a registration block rather than reconstructing the chain all over again from scratch.

In one aspect, the present disclosure relates to a registration system that is compatible with a surgical navigation system, where the surgical navigation system may include a processor and a localizer. In a first embodiment, a registration system includes a probe and an implant connectable to a bone. The probe includes a probe body and a probe tip disposed at an end of the probe body, the probe tip having an apex, a central axis extending through the apex, and an outer surface extending away from the apex. The implant has an exterior surface with a plurality of registration features extending therein, each of the registration features having an apex, a central axis extending through the apex of the registration feature, and an inner surface extending about the apex of the registration feature. A location of the apex of the probe tip within an intraoperative coordinate space is detected at any given time by the localizer and communicated to the processor. The outer surface of the probe tip and inner surface of each of the indentations are configured such that, when the probe tip is fully received in any one of the indentations, the apex of the probe tip engages the apex of the indentation such that the locations of the probe tip and indentation are coincident.

In a second embodiment, a registration system includes a probe and an implant connectable to a bone. The probe has a probe body and a first array of markers connected to the probe body, each of the markers being detectable by a localizer, i.e., a localizer of a surgical navigation system. The probe also includes a probe tip disposed at an end of the probe body, the probe tip having an apex, a central axis extending through the apex, and an outer surface extending away from the apex. The implant has an exterior surface with a plurality of indentations extending therein, each of the indentations having an apex, a central axis extending through the apex of the indentation, and an inner surface extending away from the apex of the indentation. Further, the outer surface of the probe tip and inner surface of each of the indentations are configured such that, when the probe tip is fully received in any one of the indentations, the apex of the probe tip engages the apex of the indentation such that a location of each apex is coincident.

Optionally, the registration system of the second embodiment may be configured such that the outer surface of the probe tip has a convex curvature defining a first radius of curvature extending from the central axis of the probe tip to the outer surface. Further, the inner surface of each of the indentations may have a concave curvature defining a second radius of curvature extending from the central axis of the indentation to the inner surface, the second radius of curvature being equal to the first radius of curvature. In some examples, the first radius of curvature may be 1 mm. Moreover, the registration system is configured such that the inner surface of each of the indentations is a surface of revolution extending symmetrically about the central axis of the indentation. In such a configuration, and not according to the claimed invention, the inner surface may be spherical or conical. Alternatively, and according to the claimed invention, the inner surface includes a spherical portion extending from the apex and a conical portion extending from the spherical portion.

Optionally, the registration system of the second embodiment may also include a reference tracker having a body connectable to a bone and a second array of markers connected to the body of the reference tracker. Each of the markers of the second array may be detectable by the localizer. Optionally, a depth of each indentation of the plurality of indentations of the implant, as measured from the apex of the indentation to an opening of the indentation, may be 0.3 mm to 0.5 mm. Optionally, a plane tangent to the outer surface of the probe tip may form an angle of 60 degrees relative to the central axis of the probe tip. In another optional configuration, the implant may be a patient specific implant, and a bone facing side of the implant may include a bone contacting surface that is configured to conform to a surface of the bone. In further optional configurations, the implant may include a plurality of screw holes extending through a registration side and a bone facing side of the implant, where the registration and bone facing sides are opposite each other. In still further optional configurations, the bone facing side of the implant may include a porous material configured to promote bony ingrowth.

In one aspect not according to the claimed invention, the present disclosure relates to a method of registration in a robotic or computer assisted surgical procedure. The procedure may be performed with a surgical navigation system. In a first embodiment not according to the claimed invention, a method of registration in a robotic or computer assisted surgical procedure includes: connecting a reference tracker to a bone; positioning an implant adjacent to the bone; digitizing the reference tracker, the implant, and the bone within a first intraoperative coordinate system of a surgical navigation system; performing a first registration by: determining within the first intraoperative coordinate system a first position of the bone and a first position of the implant relative to a first position of the reference tracker, and aligning the first positions of the bone, implant, and reference tracker in the first intraoperative coordinate system with corresponding first positions within a pre-operative coordinate system based on a first registration transformation, and if the reference tracker is moved relative to the bone after the aligning step, performing a second registration by: determining a second position of the implant relative to a second position of the reference tracker within a second intraoperative coordinate system, and aligning the second position of the reference tracker to a corresponding second position within the pre-operative coordinate space based on a second registration transformation and the second position of the reference tracker relative to the second position of the implant.

Optionally, the first registration transformation of the first embodiment may be a function of the first position of the reference tracker and the first position of the bone. Further, and also optionally, the second registration transformation of the first embodiment may be a function of the first positions of the reference tracker, bone, and implant. In another optional variation, the method of the first embodiment may include a step of determining a second position of the bone relative to the second position of the reference tracker within the second intraoperative coordinate system based on the first positions of the bone and the implant relative to the first position of the reference tracker and the second position of the implant relative to the second position of reference tracker. Optionally, the implant of the first embodiment may include a bone facing side and a registration side, the registration side having a plurality of indentations extending therein. Optionally, the digitizing step of the first embodiment may include detecting an array of markers connected to the reference tracker via a localizer of the surgical navigation system and touching a plurality of points on the bone and the plurality of indentations via a probe of the surgical navigation system. Optionally, the positioning step may include connecting the implant to the bone in a rigidly fixed relationship thereto. And, in yet another optional variation of the first embodiment, determining the second position of the implant may include digitizing the implant within the second intraoperative coordinate system.

In one aspect not according to the claimed invention, the present disclosure relates to a robotic or computer assisted surgical procedure. In a first embodiment not according to the claimed invention, a robotic or computer assisted surgical procedure includes: connecting a reference tracker to a bone at a first position relative thereto; registering a first position of the reference tracker and a position of the bone to a pre-operative coordinate system of a surgical navigation system; preparing the bone according to a pre-operative plan; moving the reference tracker to a second position relative to the bone; placing an implant against the bone so that an opening of the implant defines a first cutting location; registering the second position of the reference tracker and a position of the implant to the pre-operative coordinate system; and cutting the bone at the first cutting location based on the registered position of the implant and the second position of the reference tracker.

Optionally, the placing step of the first embodiment may include connecting the implant to the bone in a rigidly fixed relationship thereto. Optionally, the method of the first embodiment may include removing the implant from the bone prior to the cutting step. Optionally, the opening of the implant in the first embodiment may be a screw hole, and the cutting step may include drilling a hole in the bone. Optionally, registering the position of the implant as provided for in the first embodiment may include touching a plurality of indentations in the implant with a probe of the surgical navigation system. Optionally, each indentation of the plurality of indentations may have a shape corresponding to a tip of the probe. Optionally, the indentations may be configured such that touching the indentations includes contacting an apex of the tip of the probe with an apex of each of the indentations. Optionally, the preparing step of the first embodiment may include resecting a portion of the bone.

In a second embodiment not according to the claimed invention, a robotic or computer assisted surgical procedure includes: connecting a reference tracker to a bone; connecting a reference block to the bone; registering the reference tracker, bone, and reference block to a virtual coordinate space of a surgical navigation system based on coordinates of the bone and reference block relative to the reference tracker; and when the reference tracker is moved relative to the bone, re-registering the reference block, reference tracker, and bone to the virtual coordinate space based on coordinates of the bone and reference tracker relative to the reference block.

Optionally, the procedure of the second embodiment may include determining a chain of transformations that includes a first registration transformation based on coordinates of the reference tracker. Optionally, the re-registering step of the second embodiment may include determining a chain of transformations that includes a second registration transformation based on coordinates of the reference block.

BRIEF DESCRIPTION OF THE DRAWINGS

The features, aspects, and advantages of the present invention will become better understood with regard to the following description, appended claims, and accompanying drawings in which:

FIG. 1 is a perspective view of an implant according to an embodiment of the present disclosure.

FIG. 2A is a block diagram illustrating a computing device according to an embodiment of the present disclosure.

FIG. 2B is a block diagram illustrating data of the computing device of FIG. 2A.

FIG. 3A is a side elevational view of a virtual registration template according to a not-claimed embodiment of the present disclosure.

FIG. 3B side elevational view of the virtual registration template with a schematic representation of a registration tool according to the invention in multiple orientations.

FIG. 3C is a side elevational view of a virtual registration template according to another embodiment of the invention

FIG. 3D is a side elevational view of a virtual registration template according to a not-claimed embodiment of the present disclosure.

FIG. 4A is a flow chart illustrating of a method of making the implant of FIG. 1 according to a not-claimed embodiment of the present disclosure.

FIGS. 4B-4H further illustrating the method of making the implant of FIG. 1 not according to the claimed invention.

FIG. 5A is a schematic view of a computer assisted surgery system according to an embodiment of the present disclosure.

FIG. 5B is a perspective view of a probe and probe tracker of the computer assisted surgery system according to an embodiment of the present disclosure.

FIG. 6A illustrates an operation in a method of registration.

FIG. 6B is a flow chart illustrating a method of reregistration using implant of FIG. 1 and computer assisted surgical system of FIG. 5.

FIGS. 7A-7F illustrate a method of initial patient anatomy registration with a computer assisted surgery system not according to the claimed invention.

FIGS. 8A-8C illustrate a method of reregistration of patient anatomy using a reference block according to a not-claimed embodiment of the present disclosure.

DETAILED DESCRIPTION

As used herein, the term "proximal," when used in connection with a surgical tool or device, or components of a device, refers to the end of the device closer to the user of the device when the device is being used as intended. On the other hand, the term "distal," when used in connection with a surgical tool or device, or components of a device, refers to the end of the device farther away from the user when the device is being used as intended. However, when used in connection with the human body, the term "proximal" means closer to the heart, and the term "distal" means further from the heart. As used herein, the terms "substantially," "generally," "approximately," and "about" are intended to mean that slight deviations from absolute are included within the scope of the term so modified, such as deviations of up to 10% greater or lesser than absolute.

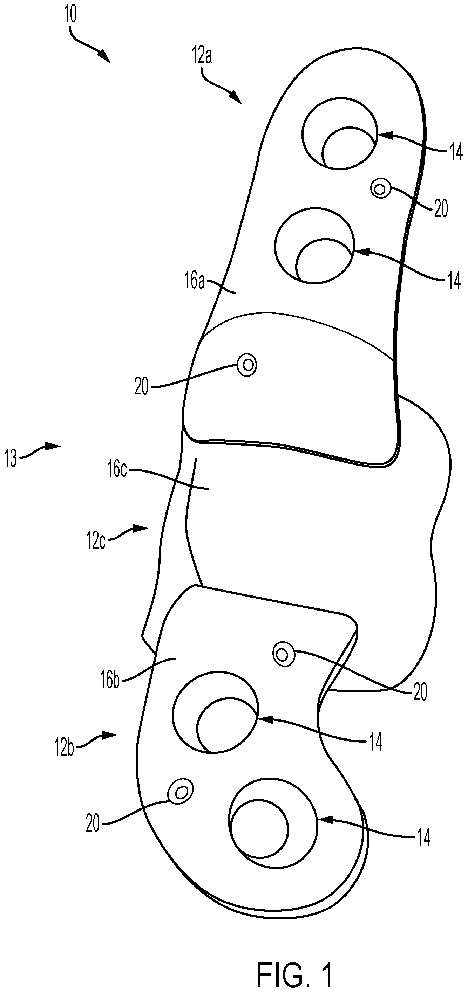

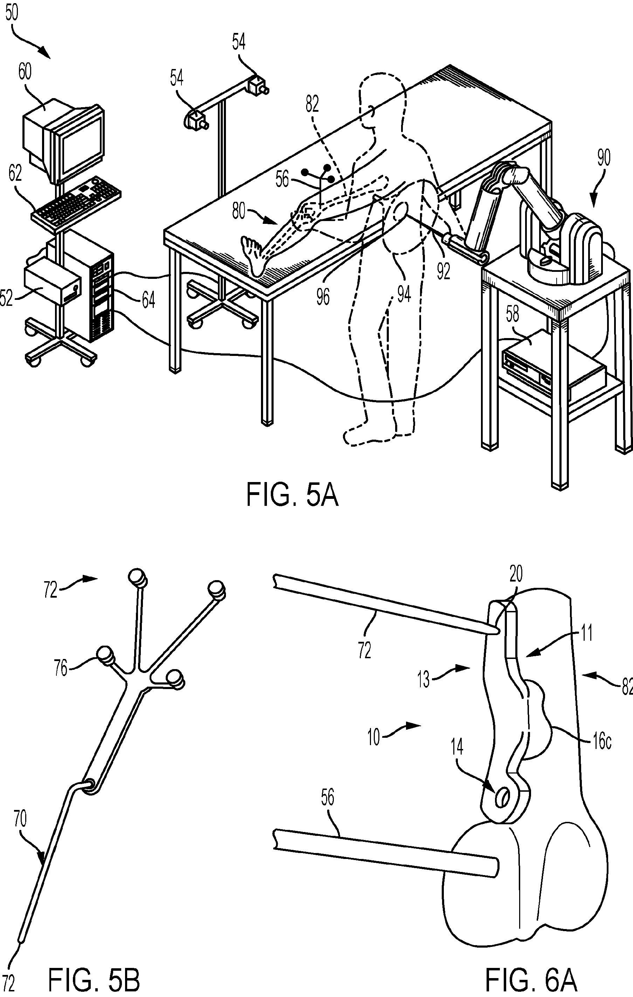

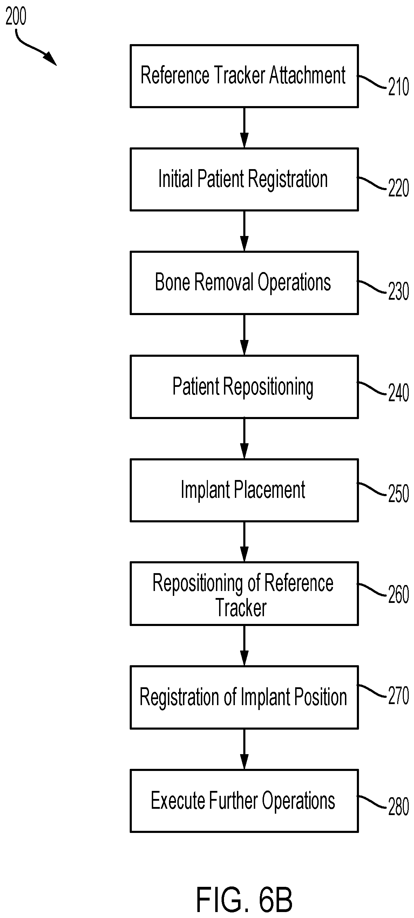

FIG. 1 depicts an implant 10 according to an embodiment of the present disclosure. Implant 10 generally includes a first side 11 and a second side 13 (see FIG. 6A). The first side or bone facing side 11 defines one or more bone facing surfaces 16a-c, and the second side or exterior side 11 defines one or more exterior surfaces 16a-c. A plurality of bone screw holes 14 extend through first and second sides 11, 13 of implant 10. However, implant 10 can have only one bone screw hole 14, for example. In addition to, or alternatively, implant 10 can have pegs, keels, or the like extending from bone facing side 11 which may be configured to be received within corresponding openings in a bone to help secure it thereto.

Second side 13 also includes a plurality of registration features or registration indentations 20 extending into the one or more exterior surfaces 16. Registration features 20, as described further below, allows implant 10 to be registered directly by a surgical navigation system so that drilling trajectories (or other paths) can be defined directly relative to implant 10. In other words, registration features 20 are located on an exterior of implant 10 and are compatible with a surgical navigation system to facilitate recognition by the surgical navigation system so that a location and orientation of implant 10 can be determined, and surgical operations can be performed on the bone based on the implant's location and orientation. To facilitate this functionality, at least three registration features 20 are provided on an exterior of implant 10, although more than three is contemplated and, in some instances, may be preferable. To the extent less than three registration features 20 are utilized, another reference, such as an axis, will be needed to ensure sufficient dimensional constraint for registration. Also, registration features 20 should generally be arranged so that they are not colinear (i.e., positioned along a single line). Furthermore, it is preferable that registration features 20 be spaced from each other so that they are not positioned directly adjacent to one another. In other words, accuracy of registration via registration features 20 generally increases the further apart the registration features are located relative to one another recognizing that implant size and various geometric features thereof, such as holes, are possible limiting factors. As an example, a minimum distance between each registration feature 20 may be 10 times tracking jitter (e.g., 1 cm depending on localizer capabilities).

Implant 10, as shown, is a patient-specific bone plate configured for augmenting a bone void formed from the removal of a bone tumor. In this regard, implant 10 includes a first portion or first plate member 12a, a second portion or second plate member 12b, and a third portion or void filling member 12c. First and second plate members 12a-b are connected to and extend outwardly from void filling member 12c, and each include at least one bone screw hole 14 extending therethrough and one or more registration features 20 on a respective exterior surface 16a-b thereof. Void filling member 12c is configured to fill a patient-specific void formed from the removal of a bone tumor and, although it is not shown, one or more registration features 20 may also be positioned on exterior surface 16c of void filling member 12c. Although implant 10, as depicted, is design with the aforementioned features specific to a case of bone tumor removal, it should be understood that any extramedullary implant or any intramedullary implant with extramedullary features can incorporate registration features 20 for surgical navigation registration, as described herein.

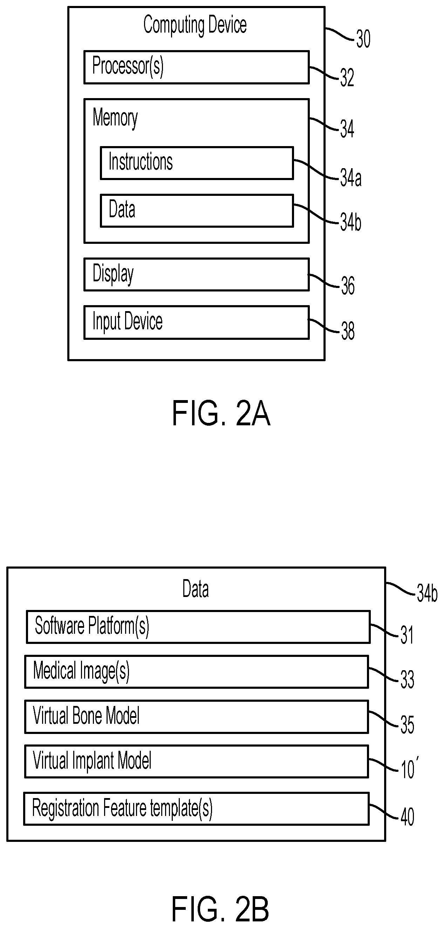

FIGs. 2A and 2B are block diagrams illustrating a computing device according to an embodiment of the present disclosure. Computing device 30 can contain one or more processors 32, memory 34, and other components typically present in general purpose computing devices, such as a display 36 and an input device 38. Display 36 can be any one of a variety of commercially available displays such as, for example, a monitor having a screen, a touch-screen, a projector, a television, or other device that is operable to display information. Additionally, input device 38 can be any commercially available devices such, as for example, mouse, keyboard, or touch screen, for example.

The one or more processors 32 can be any conventional processor, such as a commercially available CPU. Alternatively, the processors can be dedicated components such as an application specific integrated circuit ("ASIC") or other hardware-based processor. As with memory 34, the storage system thereof can be of any type of computerized storage capable of storing information accessible by computing device, such as a hard-drive, memory card, ROM, RAM, DVD, CD-ROM, write-capable, and read-only memories. Memory 34 may also include a distributed storage system where data is stored on a plurality of different storage devices which may be physically located at the same or different geographic locations.

Memory 34 can store information accessible by the one or more processors 32, including instructions 34a that can be executed by the one or more processors 32. Memory 34 can also include data 34b that can be retrieved, manipulated or stored by the processor 32. The instructions 34a can be any set of instructions to be executed directly, such as machine code, or indirectly, such as scripts, by the one or more processors. In that regard, the terms "instructions," "application," "steps," and "programs" can be used interchangeably herein. The instructions can be stored in object code format for direct processing by a processor, or in any other computing device language including scripts or collections of independent source code modules that are interpreted on demand or compiled in advance.

Data 34b may be retrieved, stored, or modified by the one or more processors 32 in accordance with the instructions 34a. For instance, although the subject matter described herein is not limited by any particular data structure, the data can be stored in computer registers, in a relational database as a table having many different fields and records, or XML documents. The data can also be formatted in any computing device-readable format such as, but not limited to, binary values, ASCII or Unicode. Moreover, the data can comprise any information sufficient to identify the relevant information, such as numbers, descriptive text, proprietary codes, pointers, references to data stored in other memories such as at other network locations, or information that is used by a function to calculate the relevant data.

As shown in FIG. 2B, data 34b may include one or more software platform 31, one or more medical image 33, a virtual bone model 35, a virtual implant model 10', and one or more virtual registration feature templates 40. Software platform 31 is configured for preoperative planning a surgical procedure. Exemplary preoperative planning software can include Stryker DesignExplorer and/or Stryker OncologyExplorer of Stryker Corporation (Kalamazoo, Michigan) and Mimics/Magics of Materialise (Leuven, Belgium). In this regard, such software platform 31 may be configured to convert, via processor 32, the one or more medical image 33 of a patient's bone into virtual bone model 35 so that certain surgical operations can be virtually performed on the virtual bone model 35 and added to the preoperative plan for later use by one or more operative software platforms configured to execute the preoperative plan. Such software platform 31 may also be configured to generate or import from a CAD platform, via processor 32, the virtual implant model 35 which may be a patient-specific virtual implant model 10' based on the virtual bone model 35 and modifications thereof via certain surgical operations applied thereto. Alternatively, virtual implant model 10' may be based on off-the-shelf implants stored in a database of memory 34 for selection by the user.

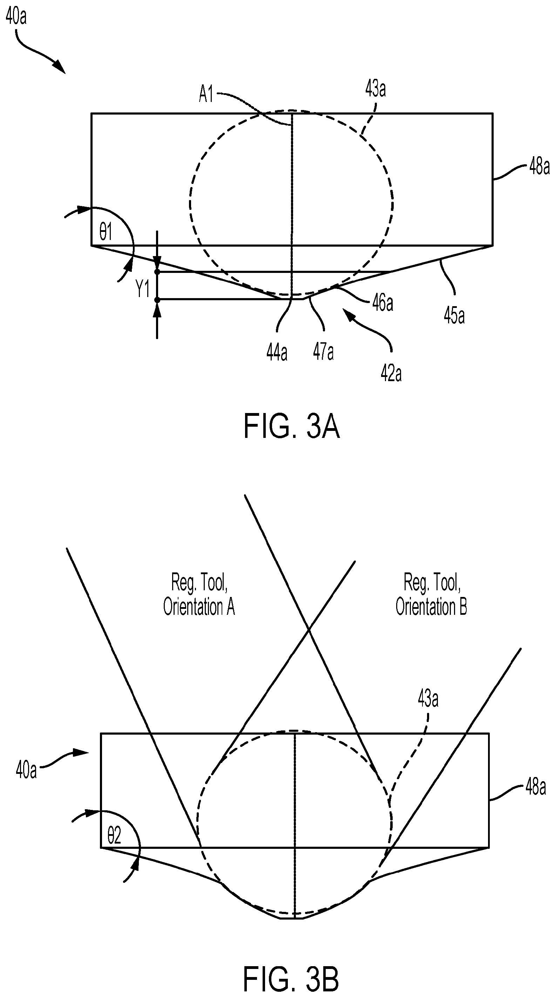

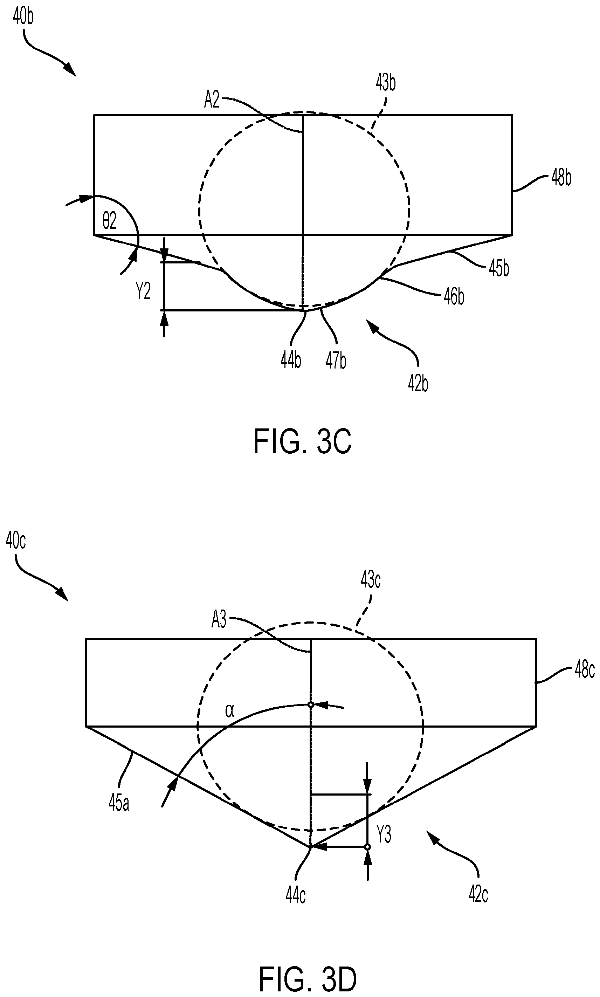

FIGs. 3A depicts a virtual registration feature template 40a according to a not-claimed embodiment of the present disclosure. As described above, registration features 20 are shown as indentations in the exterior of implant 10. In other words, registration features 20 of implant 10 are each a negative or female registration feature 20. Registration feature template 40a, on the other hand, includes a feature template face 42a that forms a positive or male registration feature which corresponds to the negative registration feature 20 of implant 10. Thus, the discussion below of the geometries of registration feature template 40a also applies to registration feature 20.

As shown, registration feature template 40a is a virtual template which may be used by software platform 31, via processor 32, to locate and form registration features 20' on virtual implant model 10' and subsequently on implant 10. Moreover, the geometries of feature template face 42a may be incorporated in a distal tip of a probe of a surgical navigation system, such as probe 70 of FIG. 5B, as discussed further below, to assist in collocating the probe tip within any one of registration features 20 for registration of implant 10.

Feature template face 42a extends from a template body 48a. Template body 48a is generally cylindrical, and template face 42a extends from template body 48a to generally form a projection (or indentation as in the case of registration features) that is symmetric relative to a template axis A1. In other words, face 42a defines an apex 44a intersected by axis A1 and is defined by a surface of revolution about axis A1 and extending from apex 44a outwardly and upwardly therefrom toward body 48a. Such surface of revolution is contoured so that a first portion 45a thereof is conical and intersects body 48a at an obtuse angle $\Theta 1$. Such angle $\Theta 1$ is selected to create a convex surface that centers sphere 43a within registration feature 20. In the embodiment depicted, $\Theta 1$ is about 105 degrees. Sphere 43a is representative of a round tip of a registration tool or probe/pointer tip. A second portion 46a is a first arcuate portion (i.e., arcuate in a plane containing axis A1) that has a radius of curvature larger than a sphere 43a bisected by the plane containing axis A1. Such sphere 43a may have a diameter of 2 mm, for example. In this regard, when sphere 43a contacts second portion 46a as shown in FIG. 3A, area-contact is formed. A third portion 47a of face 42a forms a second arcuate portion, also referred to herein as a rounded nose, that extends from first arcuate portion 46a while having a smaller radius of curvature than first arcuate portion 46a. Apex 44a is centered on and intersects nose 47a.

Template body 48a is a cylinder of constraint. In other words, template body 48a, which is depicted as a cylinder, determines an orientational boundary for a registration tool relative to a registration feature 20 that can be maintained to still be able to achieve a valid registration even if the registration tool itself is not coaxial with axis A1. This is depicted in FIG 3B in which a registration tool is shown in both a first orientation A and a second orientation B and sphere 43a depicts a registration tool tip. As shown, sphere 43a can sufficiently form area contact with second portion 46a so as to form a ring of contact about axis A1 in both orientations A and B. Therefore, in both orientations, a registration is valid. However, should the registration tool orientation extend beyond the boundary established by template body 48a, a ring of contact will not be formed, and the registration will be invalid.

As mentioned, template 40a may be used to form a registration feature 20 on implant 10 such that registration feature 20 is effectively a negative impression of face 42a. As such, template body 43a establishes a boundary of registration feature 20 with an exterior surface 16a-c of implant 10. However, registration feature 20 may not be an impression of the entirety of face 42a. For example, as shown in FIG. 3A, registration feature 20 may have a depth Y1 and therefore is an impression of only that portion of template face 42a that extends from apex 44a upwardly the linear distance Y1. In one example, Y1 may be 0.3 mm. Thus, in some embodiments, a registration feature 20 of implant 10 may only include first and second arcuate portions 46a-c. Additionally, although registration feature 20 may preferably be an indentation in an implant surface, it is contemplated that registration feature 20 may be in the form of a male projection from an implant surface with a probe having a corresponding female receiver for such male projection.

FIG. 3B depicts a virtual registration feature template 40b according to another embodiment of the present disclosure. Registration feature template 40b is similar to template 40a and is therefore accorded like reference numerals to that of template 40a. For instance, template 40b includes a template body 48a, a template face 42b extending from template body 48b, and an axis A2 extending through an apex 44b of template face 42b. Additionally, a first portion 45b of template face 42b intersects template body 48b at an angle $\Theta2$ which may be equal to angle $\Theta1$. However, feature template 40b differs in that feature depth Y2 is greater than Y1. For example, Y2 may be 0.5 mm. Additionally, first arcuate portion 46a may have a smaller radius of curvature than first arcuate portion 45a of template 40a such that template face 42b has a more bulbous appearance.

FIG. 3C depicts a virtual registration feature template 40c according to a further embodiment of the present disclosure. Registration feature template 40c is similar to template 40a and is therefore accorded like reference numerals to that of template 40a. For instance, template 40c includes a template body 48c, a template face 42c extending from template body 48c, and an axis A3 extending through an apex 44c of template face 42c. However, template face 42c is entirely conical such that when the conical surface 45a of face 42c tangent to and contacts sphere 43c, as shown in FIG. 3C, line-contact is formed.

FIG. 5 depicts a surgical navigation system 50, which is discussed further below. It is noted here that surgical navigation system 50 includes a probe 70 which can be used to register implant 10 with surgical navigation system 50. In this regard, a probe tip 72 may include geometry identical to that of any one of registration feature templates 40a-c and therefore also to that of registration feature 20. As such, the geometry of probe tip 76 can be defined by any one of the aforementioned registration templates 40a-c.

In other embodiments, probe tip 72 may not be identical to the geometry of registration feature 20 but may instead have a geometry that is operable with registration feature 20. In other words, tip 72 of probe 70 and the inner shape of the indentation formed by feature 20 may be such that probe tip 72 is always at the same position when fully disposed within a registration feature 20, within a tolerated range of orientations of probe 70 In this regard, if the angle 70 of probe is too large relative to a central axis of feature 20, then the contact between tip 72 and the inner geometry of registration feature 20 does not form circular contact and probe is no longer within the cylinder of constraint so that there is collision between probe and the cylindrical rim on the implant surface, as shown in FIG. 3B. This provides visual and tactile feedback that the probe's orientational limits relative to registration feature 20 have been reached and that any reading outside this limit is invalid. When registration features 20 are registered with system 50, the position of the tip 76 is digitized so that its location is always known within system 50. Therefore, the geometry of probe tip 76 is preferably such that it is capable of repeatably achieving the same position within a registration feature 20 regardless of which orientation probe 72 is in so that system 50 can accurately determine the location of registration feature 20 based on the location of probe tip 76. Thus, probe 72 need not be aligned with axis A1, A2, or A3, as long as probe tip 76 is full seated within registration feature 20 and contacting the inner surfaces thereof. As such, probe tip 76 may be a sphere or spherical, like that of spheres 43a-c, so that when it is disposed within a registration feature 20 and in contact therewith, the location of such registration feature 20 can be precisely determined by the location of probe tip 76.

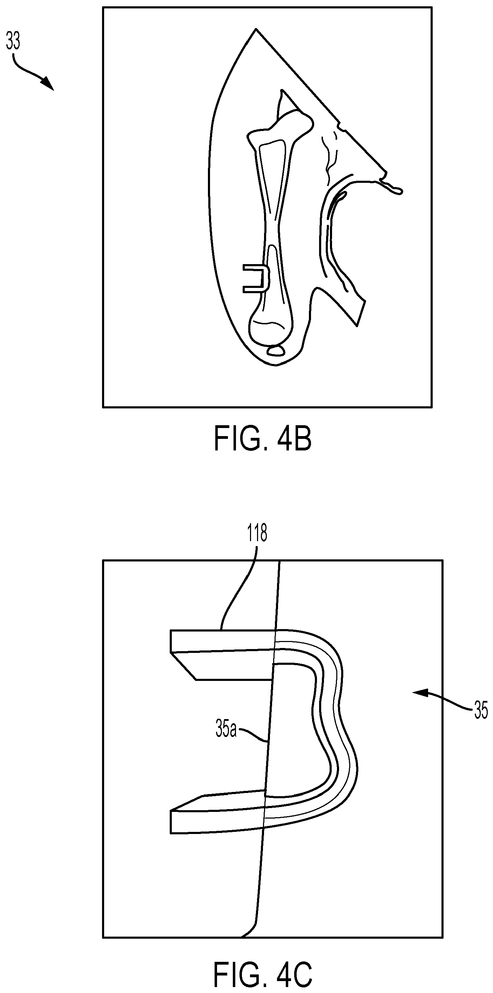

FIGs. 3A-3D is a block diagram illustrate a method of making implant 10. During a preoperative planning phase of a surgical procedure, one or more medical image 33 of a patient's affected anatomy is obtained, which in the exemplary case of the present disclosure is a femur, as shown in FIG. 4B. The medical image 33 can be acquired via a variety of medical imaging means, such as magnetic resonance imaging (MRI), computerized tomography (CT), and X-ray, for example. Software platform 31 may then then generate 110 a virtual bone model 35 from the medical image 33 according to known techniques or import the virtual bone model 35 from another software platform configured to generate the virtual bone model 35.

The virtual bone model 35 is then manipulated in a virtual coordinate space. In this regard, certain surgical operations may be performed on the virtual bone 35 in the virtual coordinate space to develop a preoperative plan 120 to be applied through the aid of a robot and/or computer during the surgical procedure. As shown in FIG. 4C, such surgical operations may include applying a resection envelope or resection boundary 118 surrounding a bone segment 35a containing a bone tumor, for example. A robot operating a cutting instrument, such as a rotating burr, for example, may follow this resection envelope 118 to remove a correspondingly shaped segment of bone from the patient during the procedure. A virtual implant 10', which may be patient-specific so that it matches the geometry of the removed segment of bone 35a, may then be generated based the resection envelope and curvatures of the cortical bone. Virtual implant 10 is based upon implant 10 which is an exemplary implant that includes void filling member 12c corresponding to a void formed by the removal of bone segment 35a and is secured in place via bone screws applied to first and second plate members 12a and 12c. However, in some embodiments, a standard implant saved in a database may be generated and applied to virtual bone 35 where applicable.

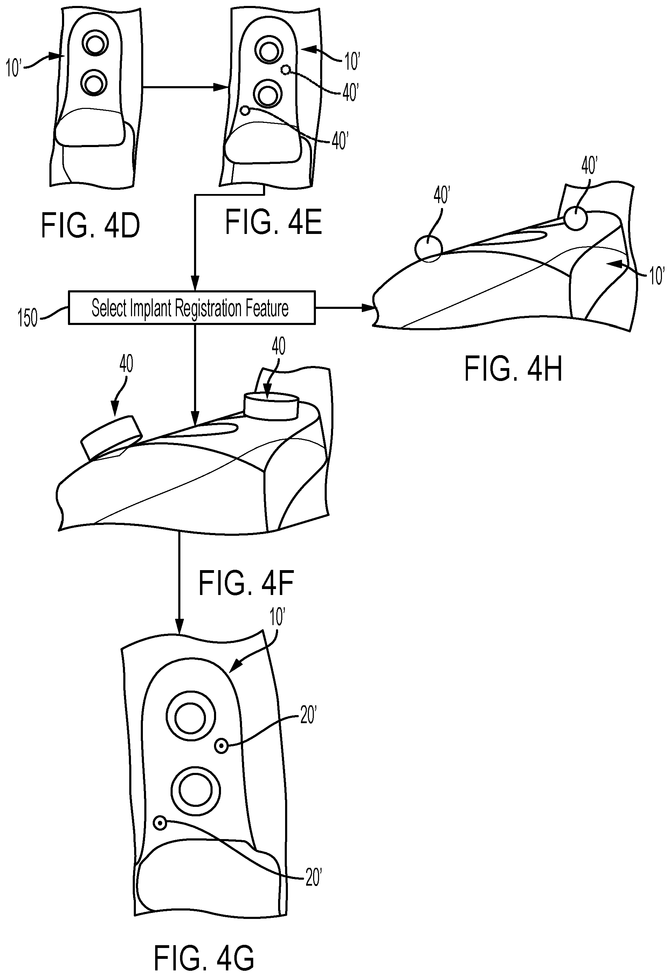

Continuing within the virtual space, the surgical plan and virtual implant 10' may be imported 130 into the software platform 31 to the extent that the surgical plan and/or virtual implant 10' were generated by another software platform or software module. Virtual implant 10' is then applied to virtual bone 35 in a manner desirable for final implantation, as shown in FIG. 4D.

At step 140, the operator may then place points or markers 40' at locations desired for registration features 20, as shown in FIG. 4E. Software platform 31 may warn the operator if such markers 40' are placed too close together or colinearly. Alternatively, software platform 31 may automatically place markers 40' at various locations on virtual implant 10' in accordance with pre-selected criteria input by the operator.

The operator then calls upon the database of registration feature templates and select 150 the desired feature template, such as templates 40a-c. Once selected, registration templates 40 appear at the locations designated by markers 40', as shown in FIG. 4F. Such templates 40 are automatically or manually oriented so that the axis of a feature face 42a-c, such as axes A1, A2, or A3, is oriented perpendicular 160 to the exterior surface of virtual implant 10' at that designated location. Registration face 42a-c of registration feature 40 is placed through the exterior surface of virtual implant 10' to a depth desired for the particular registration feature 40, such as depths Y1, Y2, or Y3 of respective templates 40a-c. Thereafter, a command may be given which subtracts 170 registration feature 20' from the virtual implant 10', as shown in FIG. 4G.

Implant 10 may then be manufactured 180 in accordance with the virtual implant model 10'. In this regard, implant 10 may be manufactured in accordance with known techniques, such as additive manufacturing, metal injection molding, casting, forging, machining, and the like so that it includes registration features 20 at the desired locations.

Software platform 31 may also generate coordinates for each registration point 190 within the virtual coordinate system and their coordinates relative to other features, such as center axes of bone screw openings 10, which may then be exported to a intraoperative software platform so that when implant 10 is registered via registration features 20, the surgical navigation system can then direct surgical operations, such as drilling into bone via a predetermined trajectory and depth.

FIG. 5A is a diagrammatic illustration of an exemplary operating room in which a haptic device 90 is used with a computer-assisted surgery system 50. Computer-assisted surgery system 50 may include a display device 60, an input device 62, and a processor-based system 64, for example a computer. Input device 62 may be any suitable input device including, for example, a keyboard, a mouse, or a touch screen. Display device 60 may be any suitable device for displaying two-dimensional and/or three-dimensional images, for example a monitor or a projector. If desired, display device 60 may be a touch screen and be used as an input device. One example of a system incorporating a haptic device 90 is described in greater detail in U.S. Patent No. 7,831,292.

Haptic device 90 is, in the illustrated example, a robotic device. Haptic device 90 may be controlled by a processor-based system, for example a computer 58. Computer 58 may also include power amplification and input/output hardware. Haptic device 90 may communicate with computer-assisted surgery system 50 by any suitable communication mechanism, whether wired or wireless.

Also shown in FIG. 5A is a storage medium 52 which may be coupled to processor-based system 64. Storage medium 52 may accept a digital medium which stores software and/or other data, such as the preoperative surgical plan. A surgical tool or instrument 92 is shown coupled to haptic device 90. Surgical tool 92 is preferably mechanically coupled to haptic device 90, such as by attaching or fastening it. However, if desired, surgical tool 92 may be coupled, either directly or indirectly, to haptic device 90 by any other suitable method, for example magnetically. Surgical tool 92 may be haptically controlled by a surgeon remotely or haptically controlled by a surgeon present in proximity to surgical tool 92, although autonomous control with surgeon oversight is possible as well. Surgical tool 92 may be, for example, a bur, saw, laser, waterjet, cautery tool, or other trackable tool capable of cutting or otherwise shaping or resecting patent tissue, including bone.

Haptic object 94 is a virtual object used to guide and/or constrain the movement and operations of a working end 96 of surgical tool 92 to a target area inside a patient's anatomy 80, for example the patient's leg. In this example, haptic object 94 is used to aid the surgeon to target and approach the intended anatomical site of the patient. Haptic feedback forces may be used to slow and/or stop the surgical tool's movement if it is detected that a portion of surgical tool 92 will intrude or cross over pre-defined boundaries of the haptic object. Furthermore, haptic feedback forces can also be used to attract (or repulse) surgical tool 92 toward (or away from) haptic object 94 and to (or away from) the target. If desired, surgeon 98 may be presented with a representation of the anatomy being operated on and/or a virtual representation of surgical tool 92 and/or haptic object 94 on display 60.

FIG. 5B a probe 70 that may be used in conjunction with computer assisted surgery system 50. In this regard, probe has a probe tip 72 configured to engage registration features 20 of implant 10 to register implant within system 50, as described above. Probe 70 may be handheld so that it is manually operable. A probe tracker 72 is connected to an end of probe 70 and is characterized by an array of fiducials 76 configured to be tracked by camera 54, as discussed further below. Alternatively, probe 70 may be connected to haptic device 90 and operated manually by the surgeon to contact registration features 20 of implant 10 for registration thereof, in which case haptic device 90 may send a signal to computer 64 regarding the location of tip 72 of probe 70 when in contact with registration features 20.

The computer-assisted surgery ("CAS") system preferably includes a localization or tracking system that determines or tracks the position and/or orientation of various trackable objects, such as surgical instruments, tools, haptic devices, patients, donor tissue and/or the like. The tracking system may continuously determine, or track, the position of one or more trackable markers disposed on, incorporated into, or inherently a part of the trackable objects, with respect to a three-dimensional coordinate frame of reference. Markers can take several forms, including those that can be located using optical (or visual), magnetic or acoustical methods. Furthermore, at least in the case of optical or visual systems, location of an object's position may be based on intrinsic features, landmarks, shape, color, or other visual appearances, that, in effect, function as recognizable markers.

Any type of tracking system may be used, including optical, magnetic, and/or acoustic systems, which may or may not rely on markers. Many tracking systems are typically optical, functioning primarily in the infrared range. They may include a stationary stereo camera pair that is focused around the area of interest and sensitive to infrared radiation. Markers emit infrared radiation, either actively or passively. An example of an active marker is a light emitting diode ("LED"). An example of a passive marker is a reflective marker, such as ball-shaped marker with a surface that reflects incident infrared radiation. Passive systems may include an infrared radiation source to illuminate the area of focus. A magnetic system may have a stationary field generator that emits a magnetic field that is sensed by small coils integrated into the tracked tools.

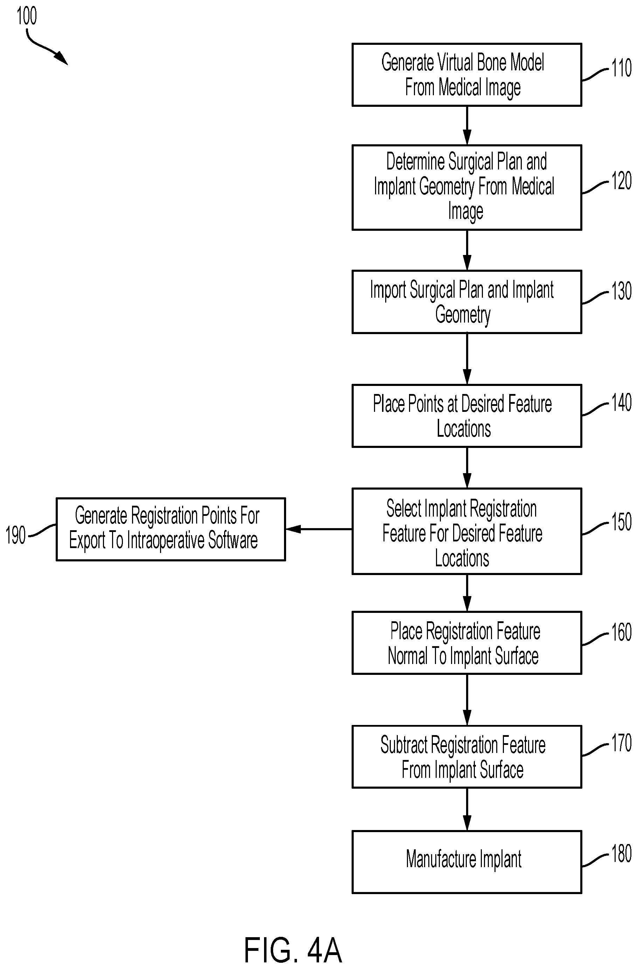

With information from the tracking system on the location of the trackable markers, CAS system 50 may be programmed to be able to determine the three-dimensional coordinates of an end point or tip of a tool and, optionally, its primary axis using predefined or known (e.g., from calibration) geometrical relationships between trackable markers on the tool and the end point and/or axis of the tool. A patient, or portions of the patient's anatomy, can also be tracked by attachment of arrays of trackable markers. In the illustrated example, the localizer is an optical tracking system that comprises one or more cameras or localizers 54 that preferably track an anatomy tracker or reference marker 56 that defines an operative coordinate space and other markers/trackers within an optical filed of localizers 54, such as probe tracker 72. As shown in FIG. 4, cameras 54 may be coupled to processor-based system 64. If desired, cameras 54 may be coupled to computer 58. Anatomy tracker 56 may be a conventional anatomy tracker. If desired, anatomy tracker may be rigidly attached to haptic device 90 or integrated into the design of haptic device 90.

In one implementation, processor-based system 64 may include image guided surgery software to provide certain user functionality, e.g., retrieval of previously saved surgical information, preoperative surgical planning, determining the position of the tip and axis of instruments, registering a patient and preoperative and/or intraoperative diagnostic image datasets to the coordinate system of the tracking system, etc. Full user functionality may be enabled by providing the proper digital medium to storage medium 52 coupled to computer 64. The digital medium may include an application specific software module. The digital medium may also include descriptive information concerning the surgical tools and other accessories. The application specific software module may be used to assist a surgeon with planning and/or navigation during specific types of procedures. For example, the software module may display predefined pages or images corresponding to specific steps or stages of a surgical procedure. At a particular stage or part of a module, a surgeon may be automatically prompted to perform certain tasks or to define or enter specific data that will permit, for example, the module to determine and display appropriate placement and alignment of instrumentation or implants or provide feedback to the surgeon. Other pages may be set up to display diagnostic images for navigation and to provide certain data that is calculated by the system for feedback to the surgeon. Instead of or in addition to using visual means, the CAS system could also communicate information in other ways, including audibly (e.g., using voice synthesis) and tactilely, such as by using a haptic interface. For example, in addition to indicating visually a trajectory for a drill or saw on the screen, a CAS system may feed information back to a surgeon whether he is nearing some object or is on course with an audible sound. To further reduce the burden on the surgeon, the module may automatically detect the stage of the procedure by recognizing the instrument picked up by a surgeon and move immediately to the part of the program in which that tool is used.

The software which resides on computer 64, alone or in conjunction with the software on the digital medium, may process electronic medical diagnostic images, register the acquired images to the patient's anatomy, and/or register the acquired images to any other acquired imaging modalities, e.g., fluoroscopy to CT, MRI, etc. If desired, the image datasets may be time variant, i.e., image datasets taken at different times may be used. Media storing the software module can be sold bundled with disposable instruments specifically intended for the procedure. Thus, the software module need not be distributed with the CAS system. Furthermore, the software module can be designed to work with specific tools and implants and distributed with those tools and implants.

Although the foregoing CAS system 50 was described as including a haptic system to provide computer assistance for an operator, such system 50 may be automated such that device 90 may operate independently without the manual manipulation by the operator. In this regard, the automated system may follow the preoperative plan with operator supervision.

In a method utilizing implant 10 with reference features 20, an anatomy reference tracker 56 is rigidly attached 210 to the bone 82. For example, as shown in FIG. 5A, anatomy tracker 56 is rigidly connected to the femur 82. Thereafter, the patient's femur 82 is initially registered 220 with system 50 so that the femur's position and orientation in the operative coordinate space corresponds to the virtual bone model 35 in the virtual coordinate space.

Once the patient's anatomy is registered, bone removal operations 230 are performed via haptic/robotic system 90. In the exemplary embodiment provided herein, a bone segment corresponding to the virtual segment 35a is removed from femur 82.

At this stage, bone screw holes need to be formed in femur 82 so that implant 10 can be secured to the bone. However, due to the positioning of implant 10 on bone 82 and screw hole trajectories relative to the working environment 94 of the haptic/robotic system 70, the patient must be repositioned 240 so that implant 10 is properly within the working envelope 74. This has the effect of disrupting the initial patient registration.

However, since implant 10 has registration features 20, system 50 can use such features 20 to guide drilling and driving operations. In this regard, implant 20 is placed 250 onto bone 82 so that void filling member 12c is positioned within the void formed in the bone 82, as shown in FIG. 6A. Reference tracker 56 is repositioned 240 and with implant 10 in its implantation position relative to bone 82, probe 70 is used to register 270 implant 10 with system 50. It is noted that the optimal intra-operative position of implant 20 after a robotic incision might slightly deviate from the pre-operatively planned position. However, screw fixation is intended to fix implant 20 in the optimal intra-operative position. Reregistration of implant 20 in the manner described herein ensures that optimal placement is achieved. In this regard, probe tip 72 engages each registration feature 20, as shown in FIG. 6A, which tells system 50 where each registration feature 20 is located relative to reference tracker 56 and correspondingly within the operative coordinate system. Based upon the preoperative data provided by software platform 31, system 50 can then determine the positioning and orientation of implant 10 and map the locations of the drilling trajectories. The haptic system/robot 90 then performs further surgical operations 280 which, in the example provided herein, includes drilling screw holes for implant 10. It is noted that, even if implant 10 is removed prior to further surgical operations, system 50 can still perform these surgical operations based in the implant registration as it can utilize reference tracker 56 to guide these operations.

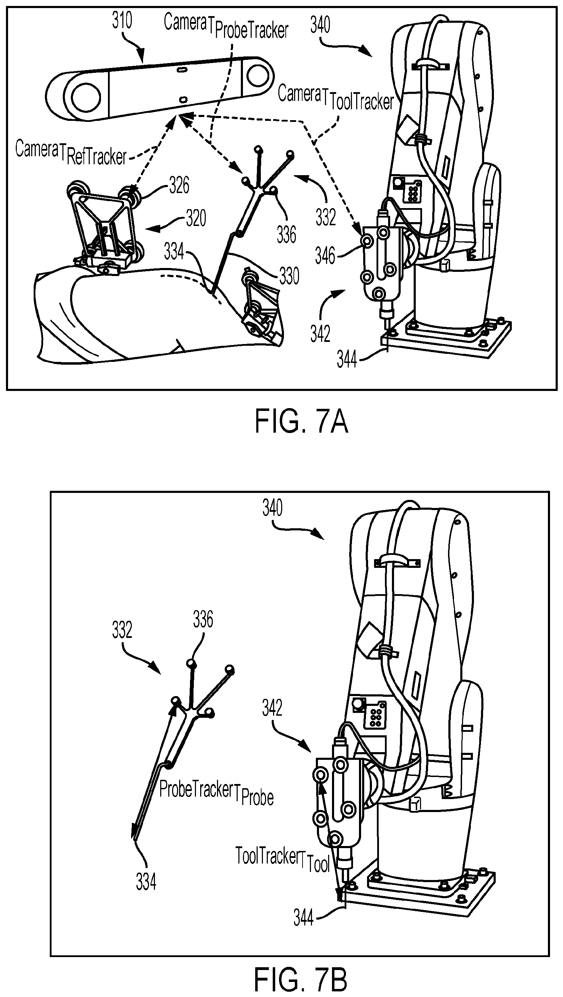

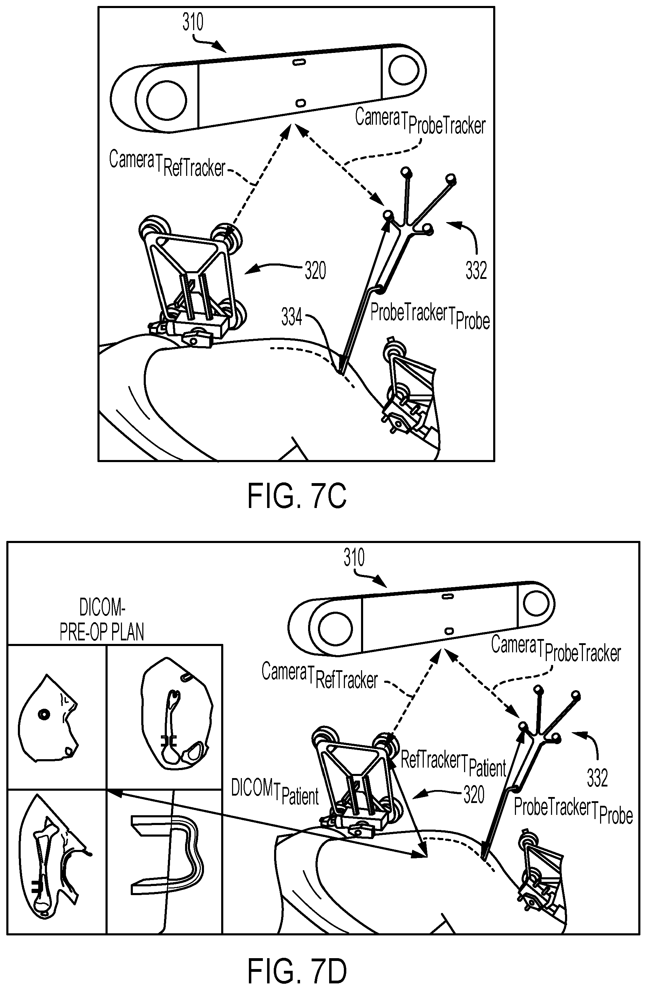

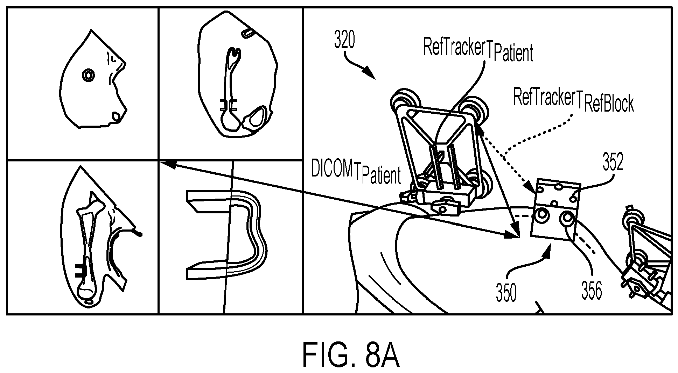

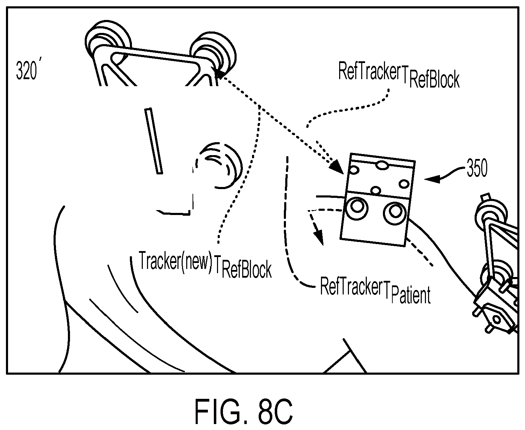

FIGS. 7A-8C depict a not-claimed embodiment of the present disclosure configured to simplify reregistration. Such embodiment includes a registration block 350 (see FIG. 8A) that is similar to implant 10 in that it includes exterior surfaces with registration features 352 located on one or more of such exterior surfaces. Such registration features 352 may be identical to registration features 20 of implant 10 in terms of geometry. However, unlike with implant 10, registration block 350 is not permanently implanted, but rather is temporarily secured to the patient's anatomy at the outset of a procedure so that in the event an initial registration of a reference tracker is invalidated due to movement of the reference tracker relative to bone, for example, registration block 350 is available to reregister the bone provided that the positioning of registration block 350 has also not been disturbed. In this regard, registration block 350 is configured to be secured to bone such as via bone pins and the like. Reference block 350 may also have fiducials 356 compatible with camera of a navigation system so that it can be detected and tracked by the camera by the navigation system, such as system 50.

In a typical registration process, as illustrated in FIGs. 7A-7F, initial registration is performed between a preoperative image coordinate space (DICOM) and an operative coordinate space. A reference tracker 320 is typically secured to the bone or anatomy and establishes the intraoperative coordinate space. As mentioned previously, any change in position or orientation of reference tracker 320 relative to the patient's actual anatomy invalidates the registration as the surgical navigation system, such as system 50, only has awareness of the patient's anatomy through reference tracker 320, as described in more detail below. Thus, changing the position or orientation of reference tracker 320 creates a break in a link of a series of transform functions that traditionally could only be repaired by conducting registration over again from scratch.

To further elaborate, in a typical initial registration, a series of registration transforms are synchronized to complete registration. FIG. 7A illustrates a transform involving a camera 310, reference tracker 320 (RefTracker), a probe 330, a probe tracker 332 (ProbeTracker), a surgical tool 344, and a tool tracker 342 (ToolTracker). Camera or localizer 310 is a part of a navigation system, such as system 50, which may include a robot 340 that holds and manipulates surgical tool 344 in accordance with a preoperative plan. Reference tracker 320, probe tracker 332, and tool tracker 342 each have an array of reflectors or fiducials 326, 336, 346 that are compatible with camera 310 so that the navigation system can determine the precise locations of objects connected thereto such as the patient's anatomy, probe 330, and tool 344, respectively. The following transform expresses the relationship between reference tracker 320 and probe 330 so that the location of a probe tip 334 relative to the reference tracker 320 can be determined.

$$^{\text{RefTracker}}T_{\text{probe}} = {}^{\text{Camera}}T_{\text{RefTracker}}{}^{-1} \text{ X } {}^{\text{Camera}}T_{\text{ProbeTracker}} \text{ X } {}^{\text{ProbeTracker}}T_{\text{Probe}}$$

Assuming the probe and tool trackers 332, 342 have been calibrated relative to the probe 330 and tool 344, respectively, as shown in FIG. 7B, probe tip 334 can then be used by the operator to contact designated points on the bone thereby registering the bone anatomy relative to reference tracker 320, as illustrated in FIG. 7C.

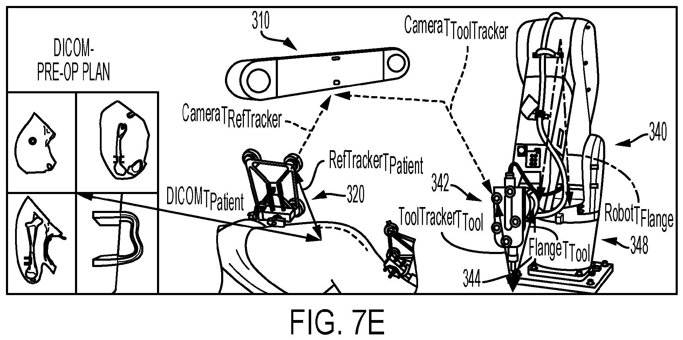

In order to follow the preoperative surgical plan, the preoperative coordinate system (DICOM) must be aligned with the intraoperative coordinate system (RefTracker) through the registration process, as illustrated in FIG. 7E. In this regard, points collected in the intraoperative coordinate system ($^{\text{RefTracker}}T_{\text{Patient}}$) are aligned with the same points in the DICOM coordinate system ($^{\text{Dicom}}T_{\text{patient}}$) to give the registration transform:

$$^{\text{DICOM}}T_{\text{RefTracker}} = {}^{\text{DICOM}}T_{\text{Patient}} \text{ X } {}^{\text{RefTracker}}T_{\text{patient}}{}^{-1}$$

One can therefore track the position of probe 330 in the original DICOM coordinate system by linking the above transformations.

$$^{\text{DICOM}}T_{\text{Probe}} = {}^{\text{DICOM}}T_{\text{Patient}} \text{ X } {}^{\text{RefTracker}}T_{\text{Patient}}{}^{-1} \text{ X } {}^{\text{Camera}}T_{\text{RefTracker}}{}^{-1} \text{ X } {}^{\text{Camera}}T_{\text{ProbeTracker}} \text{ X }$$
$$^{\text{ProbeTracker}}T_{\text{Probe}}$$

In order for robot 340 to follow a path defined in DICOM, two additional transformations are typically needed. The first is a calibration of tool 334 to the last joint or flange 348 of the robot ($^{\text{Flange}}T_{\text{Tool}}$) and then a transformation back to the base coordinate system of the robot ($^{\text{Robot}}T_{\text{Flange}}$) that is given by the kinematic model of the robotic arm, as shown in FIG. 7E. Transforming the position of robot 340 to the DICOM coordinate system results then in the following transformation chain:

$$^{\text{DICOM}}T_{\text{Robot}} = {}^{\text{DICOM}}T_{\text{Patient}} \text{ X } {}^{\text{RefTracker}}T_{\text{Patient}}{}^{-1} \text{ X } {}^{\text{Camera}}T_{\text{RefTracker}}{}^{-1} \text{ X } {}^{\text{Camera}}T_{\text{ToolTracker}} \text{ X }$$
$$^{\text{ToolTracker}}T_{\text{Tool}} \text{ X } {}^{\text{Flange}}T_{\text{Tool}}{}^{-1} \text{ X } {}^{\text{Robot}}T_{\text{Flange}}{}^{-1}$$

By inverting this transformation (i.e., $^{\text{Robot}}T_{\text{Dicom}} = {}^{\text{DICOM}}T_{\text{Robot}}{}^{-1}$) it allows a tool path defined in DICOM to be transformed to the robot base coordinate system so that robot 340 is synchronized with DICOM and therefore robot 340 can accurately follow the preoperative plan.

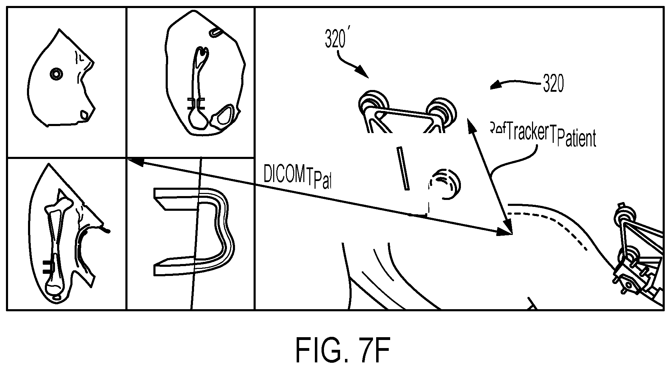

However, if reference tracker 320 is moved relative to the bone for any reason, such as illustrated in FIG. 7F in which reference tracker 320 is moved from a first position to a second position 320' relative to the patient's anatomy, the $^{\text{RefTracker}}T_{\text{Patient}}$ transformation is no longer valid as the registration transformation chain is broken and the registration transformation is lost. Until now, the only way to recover the registration was to build up the chain again from scratch by requiring all of the points and surfaces on the patient's anatomy in the intraoperative coordinate space and then recompute the transformation with no guarantee of accuracy or consistency.

Reference block 350, as shown in FIGs. 8A-8C, provides a solution to this problem in that it allows the link to be repaired without the need to perform registration all over again from scratch. In this regard, reference block 350 is secured to the patient's anatomy at a location offset from reference tracker 320 and is digitized at the outset of the procedure using probe 330 to contact reference features 352, as described above with respect to implant 10. A transformation chain between reference block and DICOM ($^{\text{DICOM}}T_{\text{RefBlock}}$) is then created, as shown in FIG. 8A, which includes a transform between reference tracker 320 and reference block 350:

$$^{\text{DICOM}}T_{\text{RefBlock}} = {}^{\text{DICOM}}T_{\text{Patient}} \text{ X } {}^{\text{RefTracker}}T_{\text{Patient}}{}^{-1} \text{ X } {}^{\text{RefTracker}}T_{\text{RefBlock}}$$

This transformation is a static registration transformation (assuming that reference block 350 is rigidly fixed and does not move relative to the bone) allowing the registration to be recovered in the event that the transform $^{RefTracker}T_{Patient}$ is lost.

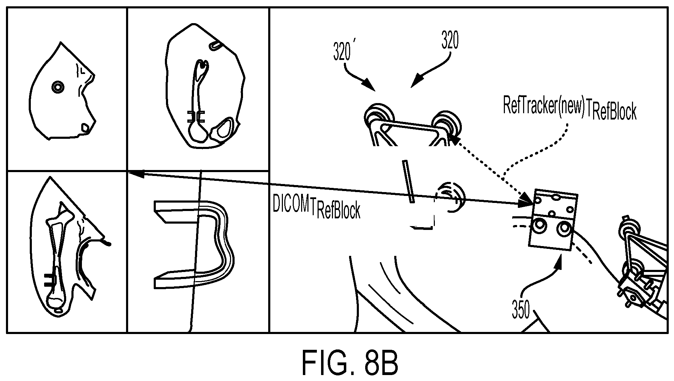

In this regard, registration can be recovered by re-digitizing reference block 350 and re-computing the transform between reference tracker 320', in its new position, and reference block 350 ($^{RefTracker(new)}T_{RefBlock}$). As illustrated in FIG. 8B, this transform between reference tracker 320' and reference block 350 then allows the new position of reference tracker 320' to be obtained relative to DICOM:

$$^{DICOM}T_{RefTracker(new)} = {^{DICOM}T_{RefBlock}} \; X \; {^{RefTracker(New)}T_{RefBlock}}^{-1}$$

The computation of the transformation between the reference tracker and patient ($^{RefTracker}T_{Patient}$) can therefore be obtained without needing to explicitly re-digitize the patient surfaces/points. Thus, the registration transformation that results is

$$^{RefTracker(New)}T_{Patient} = {^{RefTracker(new)}T_{RefBlock}} \; X \; {^{RefTracker}T_{RefBlock}}^{-1} \; X \; {^{RefTracker}T_{Patient}}$$

where

$$^{RefTracker(new)}T_{RefBlock} \; X \; {^{RefTracker}T_{RefBlock}}^{-1}$$

represents the transformation from the old intraoperative coordinate system (RefTracker) to the new intraoperative coordinate system (RefTracker(new)). Thus, if the chain of transformations is broken, such as through inadvertent movement of the reference tracker, for example, the broken link in that chain can be repaired by using a registration block rather than reconstructing the chain all over again from scratch.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A registration system compatible with a surgical navigation system (50) having a processor (32) and a localizer (54) connected to the processor (32), comprising:

   a probe (70) having a probe body and a first array of markers connected to the probe body, each of the markers being detectable by the localizer (54), the probe (70) having a probe tip (72) disposed at an end of the probe body, the probe tip (72) having an apex, a central axis extending through the apex, and an outer surface extending away from the apex; and

   an implant (10) being connectable to a bone and having an exterior surface (16a-c), the exterior surface (16a-c) having a plurality of indentations (20) extending therein, each of the indentations (20) having an apex (44a, 44b, 44c), a central axis (A1, A2, A3) extending through the apex (44a, 44b, 44c) of the indentation (20), and an inner surface (45a, 46a, 47a, 45b, 46b) extending away from the apex (44a, 44b, 44c) of the indentation (20), the inner surface (45a, 46a, 47a, 45b, 46b) of each of the indentations (20) being a surface of revolution (43a, 43b, 43c) extending symmetrically about the central axis (A1, A2, A3) of the indentation (20) and including a spherical portion (46b) extending from the apex (44a, 44b, 44c) and a conical portion (45b) extending from the spherical portion (46b),

   wherein the outer surface of the probe tip (72) and inner surface (45a, 46a, 47a, 45b, 46b) of each of the indentations (20) are configured such that, when the probe tip (72) is fully received in any one of the indentations (20), the apex of the probe tip (72) engages the apex (44a, 44b, 44c) of the indentation (20) such that a location of each apex is coincident.

2. The system of claim 1, wherein the outer surface of the probe tip (72) has a convex curvature defining a first radius of curvature extending from the central axis of the probe tip (72) to the outer surface.

3. The system of claim 2, wherein the inner surface (45a, 46a, 47a, 45b, 46b) of each of the indentations (20) has a concave curvature defining a second radius of curvature extending from the central axis (A1, A2, A3) of the indentation (20) to the inner surface (45a, 46a, 47a, 45b, 46b), the second radius of curvature being equal to the first radius of curvature.

**4.** The system of any one of claims 2-3, wherein the first radius of curvature is 1 mm.

**5.** The system of any one of claims 1-4, further comprising a reference tracker (56) having a body connectable to a bone and a second array of markers connected to the body, each of the markers of the second array being detectable by the localizer (54).

**6.** The system of any one of claims 1-5, wherein a depth (Y1, Y2, Y3) of each indentation (20) as measured from the apex (44a, 44b, 44c)of the indentation (20) to an opening of the indentation (20) is 0.3 mm to 0.5 mm.

**7.** The system of any one of claims 1-6, wherein a plane tangent to the outer surface of the probe tip (72) forms an angle of 60 degrees relative to the central axis of the probe tip (72).

**8.** The system of any one of claims 1-7, wherein the implant (10) is a patient specific implant, and a bone facing side (11) of the implant (10) includes a bone contacting surface that is configured to conform to a surface of the bone.

**9.** The system of any one of claims 1-7, wherein the implant (10) includes a plurality of screw holes (14) extending through a registration side (13) and a bone facing side (11) of the implant (10), the registration side being opposite the bone facing side (11).

**10.** The system of any one of claims 1-7, wherein a bone facing side (11) of the implant (10) includes a porous material configured to promote bony ingrowth.


**Patentansprüche**

**1.** Registrierungssystem, das mit einem chirurgischen Navigationssystem (50) kompatibel ist, aufweisend einen Prozessor (32) und einen mit dem Prozessor (32) verbundenen Lokalisierer (54), umfassend:

eine Sonde (70) mit einem Sondenkörper und einer ersten Anordnung von Markierungen, die mit dem Sondenkörper verbunden sind, wobei jede der Markierungen durch den Lokalisierer (54) erkennbar ist, wobei die Sonde (70) eine Sondenspitze (72) aufweist, die an einem Ende des Sondenkörpers angeordnet ist, wobei die Sondenspitze (72) einen Scheitelpunkt, eine sich durch den Scheitelpunkt erstreckende Mittelachse und eine sich von dem Scheitelpunkt weg erstreckende äußere Fläche bzw. Oberfläche aufweist; und
ein Implantat (10), das mit einem Knochen verbindbar ist und eine Außenfläche bzw. -oberfläche (16a-c) aufweist, wobei die Außenfläche (16a-c) eine Mehrzahl von Einkerbungen (20) aufweist, die sich darin erstrecken, wobei jede der Einkerbungen (20) einen Scheitelpunkt (44a, 44b, 44c), eine sich durch den Scheitelpunkt (44a, 44b, 44c) der Einkerbung (20) erstreckende Mittelachse (A1, A2, A3) und eine sich von dem Scheitelpunkt (44a, 44b, 44c) der Einkerbung (20) weg erstreckende innere Fläche bzw. Oberfläche (45a, 46a, 47a, 45b, 46b) aufweist, wobei die innere Fläche (45a, 46a, 47a, 45b, 46b) jeder Einkerbung (20) eine Umdrehungs- bzw. Rotationsfläche bzw. -oberfläche (43a, 43b, 43c) ist, die sich symmetrisch um die Mittelachse (A1, A2, A3) der Einkerbung (20) erstreckt und einen sphärischen Abschnitt (46b), der sich von dem Scheitelpunkt (44a, 44b, 44c) erstreckt, und einen konischen Abschnitt (45b) beinhaltet, der sich von dem sphärischen Abschnitt (46b) erstreckt,
wobei die äußere Fläche der Sondenspitze (72) und die innere Fläche (45a, 46a, 47a, 45b, 46b) jeder der Einkerbungen (20) so konfiguriert sind, dass, wenn die Sondenspitze (72) vollständig in einer der Einkerbungen (20) aufgenommen ist, der Scheitelpunkt der Sondenspitze (72) mit dem Scheitelpunkt (44a, 44b, 44c) der Einkerbung (20) derart in Eingriff ist, dass eine Stelle jedes Scheitelpunkts übereinstimmt.

**2.** System nach Anspruch 1, wobei die äußere Fläche der Sondenspitze (72) eine konvexe Krümmung aufweist, die einen ersten Krümmungsradius definiert, der sich von der Mittelachse der Sondenspitze (72) zu der äußeren Fläche erstreckt.

**3.** System nach Anspruch 2, wobei die innere Fläche (45a, 46a, 47a, 45b, 46b) jeder Einkerbung (20) eine konkave Krümmung aufweist, die einen zweiten Krümmungsradius definiert, der sich von der Mittelachse (A1, A2, A3) der Einkerbung (20) zu der inneren Fläche (45a, 46a, 47a, 45b, 46b) erstreckt, wobei der zweite Krümmungsradius gleich dem ersten Krümmungsradius ist.

**4.** System nach einem der Ansprüche 2-3, wobei der erste Krümmungsradius 1 mm beträgt.

5. System nach einem der Ansprüche 1-4, ferner umfassend einen Referenztracker (56), der einen mit einem Knochen verbindbaren Körper und eine zweite Anordnung von Markierungen aufweist, die mit dem Körper verbunden sind, wobei jede Markierung der zweiten Anordnung durch den Lokalisierer (54) erkennbar ist.

6. System nach einem der Ansprüche 1-5, wobei eine Tiefe (Y1, Y2, Y3) jeder Einkerbung (20), gemessen von dem Scheitelpunkt (44a, 44b, 44c) der Einkerbung (20) zu einer Öffnung der Einkerbung (20), 0,3 mm bis 0,5 mm beträgt.

7. System nach einem der Ansprüche 1-6, wobei eine die äußere Fläche der Sondenspitze (72) tangierende Ebene einen Winkel von 60 Grad relativ zu der Mittelachse der Sondenspitze (72) bildet.

8. System nach einem der Ansprüche 1-7, wobei das Implantat (10) ein patientenspezifisches Implantat ist und eine dem Knochen zugewandte Seite (11) des Implantats (10) eine Knochenkontaktfläche bzw. - oberfläche beinhaltet, die konfiguriert ist, mit einer Fläche bzw. Oberfläche des Knochens konform zu sein.

9. System nach einem der Ansprüche 1-7, wobei das Implantat (10) eine Mehrzahl von Schraubenlöchern (14) beinhaltet, die sich durch eine Registrierungsseite (13) und eine dem Knochen zugewandte Seite (11) des Implantats (10) erstrecken, wobei die Registrierungsseite der dem Knochen zugewandten Seite (11) gegenüberliegt bzw. entgegengesetzt ist.

10. System nach einem der Ansprüche 1-7, wobei eine dem Knochen zugewandte Seite (11) des Implantats (10) ein poröses Material beinhaltet, das konfiguriert ist, ein Einwachsen von Knochen zu fördern.

**Revendications**

1. Un système d'enregistrement compatible avec un système de navigation chirurgicale (50) ayant un processeur (32) et un localisateur (54) connecté au processeur (32), comprenant :

   une sonde (70) ayant un corps de sonde et un premier réseau de marqueurs reliés au corps de sonde, chacun des marqueurs étant détectable par le localisateur (54), la sonde (70) ayant une pointe de sonde (72) disposée à une extrémité du corps de sonde, la pointe de sonde (72) ayant un sommet, un axe central s'étendant à travers le sommet, et une surface extérieure s'étendant à l'écart du sommet ; et
   un implant (10) pouvant être relié à un os et ayant une surface extérieure (16a-c), la surface extérieure (16a-c) ayant une pluralité d'indentations (20) s'étendant dans celle-ci, chacune des indentations (20) ayant un sommet (44a, 44b, 44c), un axe central (A1, A2, A3) s'étendant à travers le sommet (44a, 44b, 44c) de l'indentation (20), et une surface intérieure (45a, 46a, 47a, 45b, 46b) s'étendant à l'écart du sommet (44a, 44b, 44c) de l'indentation (20), la surface intérieure (45a, 46a, 47a, 45b, 46b) de chacune des indentations (20) étant une surface de révolution (43a, 43b, 43c) s'étendant symétriquement autour de l'axe central (A1, A2, A3) de l'indentation (20) et comprenant une partie sphérique (46b) s'étendant à partir du sommet (44a, 44b, 44c) et une partie conique (45b) s'étendant à partir de la partie sphérique (46b),
   dans lequel la surface extérieure de la pointe de la sonde (72) et la surface intérieure (45a, 46a, 47a, 45b, 46b) de chacune des indentations (20) sont configurées de telle sorte que, lorsque la pointe de la sonde (72) est entièrement reçue dans l'une quelconque des indentations (20), le sommet de la pointe de la sonde (72) s'engage dans le sommet (44a, 44b, 44c) de l'indentation (20) de telle sorte qu'un emplacement de chaque sommet est coïncidant.

2. Le système de la revendication 1, dans lequel la surface extérieure de la pointe de la sonde (72) présente une courbure convexe définissant un premier rayon de courbure s'étendant à partir de l'axe central de la pointe de la sonde (72) vers la surface extérieure.

3. Le système de la revendication 2, dans lequel la surface intérieure (45a, 46a, 47a, 45b, 46b) de chacune des indentations (20) présente une courbure concave définissant un second rayon de courbure s'étendant de l'axe central (A1, A2, A3) de l'empreinte (20) à la surface intérieure (45a, 46a, 47a, 45b, 46b), le second rayon de courbure étant égal au premier rayon de courbure.

4. Le système de l'une quelconque des revendications 2-3, dans lequel le premier rayon de courbure est de 1 mm.

5. Le système de l'une quelconque des revendications 1 à 4, comprenant en outre un suiveur de référence (56) dont le corps peut être relié à un os et un second réseau de marqueurs relié au corps, chacun des marqueurs du second réseau pouvant être détecté par le localisateur (54).

**6.** Le système de l'une quelconque des revendications 1 à 5, dans lequel une profondeur (Y1, Y2, Y3) de chaque indentation (20), mesurée à partir du sommet (44a, 44b, 44c) de l'indentation (20) jusqu'à une ouverture de l'indentation (20), est comprise entre 0,3 mm et 0,5 mm.

**7.** Le système de l'une quelconque des revendications 1 à 6, dans lequel un plan tangent à la surface extérieure de la pointe de la sonde (72) forme un angle de 60 degrés par rapport à l'axe central de la pointe de la sonde (72).

**8.** Le système de l'une quelconque des revendications 1 à 7, dans lequel l'implant (10) est un implant spécifique au patient, et une face orientée vers l'os (11) de l'implant (10) comprend une surface de contact avec l'os qui est configurée pour se conformer à une surface de l'os.

**9.** Le système de l'une quelconque des revendications 1 à 7, dans lequel l'implant (10) comprend une pluralité de trous de vis (14) s'étendant à travers un côté d'enregistrement (13) et une face orientée vers l'os (11) de l'implant (10), le côté d'enregistrement étant opposé à la face orientée vers l'os (11).

**10.** Le système de l'une quelconque des revendications 1 à 7, dans lequel une face orientée vers l'os (11) de l'implant (10) comprend un matériau poreux configuré pour favoriser la croissance osseuse.

FIG. 1

FIG. 2A

FIG. 2B

FIG. 3A

FIG. 3B

18

FIG. 3C

FIG. 3D

100

Generate Virtual Bone Model From Medical Image —— 110

Determine Surgical Plan and Implant Geometry From Medical Image —— 120

Import Surgical Plan and Implant Geometry —— 130

Place Points at Desired Feature Locations —— 140

Generate Registration Points For Export To Intraoperative Software —— 190

Select Implant Registration Feature For Desired Feature Locations —— 150

Place Registration Feature Normal To Implant Surface —— 160

Subtract Registration Feature From Implant Surface —— 170

Manufacture Implant —— 180

FIG. 4A

FIG. 4B

FIG. 4C

FIG. 4D

FIG. 4E

FIG. 4H

Select Implant Registration Feature

FIG. 4F

FIG. 4G

FIG. 5A

FIG. 5B

FIG. 6A

*200*

```
Reference Tracker Attachment ─── 210
          │
          ▼
Initial Patient Registration ─── 220
          │
          ▼
Bone Removal Operations ─── 230
          │
          ▼
Patient Repositioning ─── 240
          │
          ▼
Implant Placement ─── 250
          │
          ▼
Repositioning of Reference
Tracker ─── 260
          │
          ▼
Registration of Implant Position ─── 270
          │
          ▼
Execute Further Operations ─── 280
```

FIG. 6B

FIG. 7A

FIG. 7B

**FIG. 7C**

**FIG. 7D**

FIG. 7E

FIG. 7F

FIG. 8A

FIG. 8B

FIG. 8C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021243241 A1
- US 7831292 B